# EUROPEAN PATENT SPECIFICATION

(11) **EP 3 996 668 B1**
(45) Date of publication and mention of the grant of the patent: **16.10.2024**
(21) Application number: 20733469.9
(22) Date of filing: 23.06.2020
(51) Int. Cl.: A61K 8/67, A61K 8/60, A61Q 19/08

(54) **TOPICAL COMPOSITIONS AND METHODS OF USING SAME AGAINST MITOCHONDRIAL FRAGMENTATION**
TOPISCHE ZUSAMMENSETZUNGEN UND VERFAHREN ZUR VERWENDUNG DAVON GEGEN MITOCHONDRIALE FRAGMENTIERUNG
COMPOSITIONS TOPIQUES ET LEURS PROCÉDÉS D'UTILISATION CONTRE LA FRAGMENTATION MITOCHONDRIALE

(30) Priority: 12.07.2019 EP 19186086
(43) Date of publication of application: 18.05.2022
(73) Proprietor: Unilever Global IP Limited, Wirral, Merseyside CH62 4ZD (GB); Unilever IP Holdings B.V., 3013 AL Rotterdam (NL)
(72) Inventor: ROCHA, Sheila, Alves, Trumbull, CT Connecticut 06611 (US); CHIANG, Chung-Yi, Irvine, California 92602 (US); ROSA, Jose, Guillermo, Trumbull, CT Connecticut 06611 (US); NIP, John, Chun-Sing, Trumbull, CT Connecticut 06611 (US)
(74) Representative: James, Helen Sarah
(86) International application number: PCT/EP2020/067465
(87) International publication number: WO 2021/008822

(56) References cited:
- NZ-A- 526 350
- US-A- 4 956 173
- US-A- 5 939 082
- US-A1- 2004 043 013
- US-A1- 2004 047 896
- US-A1- 2004 213 819
- US-A1- 2005 095 233
- US-A1- 2006 069 059
- JEON B R ET AL: "S-adenosylmethionine protects post-ischemic mitochondrial injury in rat liver", JOURNAL OF HEPATOLOGY, ELSEVIER, AMSTERDAM, NL, vol. 34, no. 3, 1 March 2001 (2001-03-01), pages 395 - 401, XP027303154, ISSN: 0168-8278, [retrieved on 20010301]
- LOTKOVA H ET AL: "Protective effect of S-adenosylmethionine on cellular and mitochondrial membranes of rat hepatocytes against tert-butylhydroperoxide-induced injury in primary culture", CHEMICO-BIOLOGICAL INTERACTIONS, ELSEVIER SCIENCE IRLAND, IR, vol. 156, no. 1, 10 September 2005 (2005-09-10), pages 13 - 23, XP027648382, ISSN: 0009-2797, [retrieved on 20050910]

## Description

### Field of the invention

The present invention is directed to methods and uses as defined in the appended set of claims that involve topical compositions including cooperative combinations of niacinamide and S-adenosyl-L-methionine useful against mitochondrial fragmentation within skin cells.

### Background of the invention

When consumers wish to look younger by reducing facial lines, wrinkles, and blotchy marks on the skin, they find it desirable to deliver skin benefits via methods that rely on the application of topical compositions. Active ingredients for incorporation in topical compositions, which can deliver consumer skin benefits are always sought. There is an ongoing need for active ingredients for incorporation in topical compositions, which active ingredients deliver skin benefits by protecting the integrity and function of cellular components.

However, the art of leveraging the functional components of the cell to achieve youth or its perception via appearance is uncertain. While true in all cells of the body, it is particularly unpredictable whether or how cellular manipulation can achieve youthful looking skin. Important functional components of the cell are mitochondria.

Inside the human cell, oxidative phosphorylation takes place inside the mitochondria - an essential cellular component where up to 83% of the cellular energy is produced in the form of adenosine triphosphate (ATP). The cellular energy stored in ATP is then used to drive multiple essential biological processes within the human cell and by extension within the body of a mammal, including a human.

During mitochondrial oxidative phosphorylation, a low basal level of reactive oxygen species (ROS - highly reactive intermediates capable of damaging cellular components fatty acids, proteins and DNA leading to cell degeneration and death) are generated. This low level of ROS can be efficiently eradicated by constitutive antioxidant enzymes, ROS scavengers, repair enzymes and removal of damaged components. In contrast, conditions that allow for increased and sustained levels of ROS result in mitochondrial fragmentation ("MF") or dysfunctional mitochondria unable to meet the energy demands required for normal cellular functioning, particularly under stressful situations. As long-term solar ultra-violet ("UV") radiation is one of the key conditions leading to high levels of ROS, mitochondrial fragmentation is especially problematic for unprotected skin from UV radiation that causes photoaging. Mitochondrial fragmentation is also a concern during normal skin aging where repair cellular mechanisms begin to slow down. In fact, in vivo studies have shown that keratinocytes of old human skin have a significantly more fragmented mitochondrial network compared to keratinocytes of young human skin (Mellem D, Sattler M, Pagel-Wolff S, Jaspers S, Wenck H, Ru 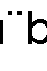 bhausen MA, et al. (2017) Fragmentation of the mitochondrial network in skin in vivo. PLoS ONE 12(6): e0174469).

Therefore, sought are technologies (compounds) that can protect the skin against mitochondrial fragmentation, maintain a basal level of reactive oxygen species, and prevent premature skin aging.

Without wishing to be bound by theory, Applicant believes that compounds that protect the skin cells from mitochondrial fragmentation contribute to the increase in cellular energy required to maintain or improve the integrity and function of the skin. Boosting amounts of such compounds in skin cells is associated with skin benefits (young skin phenotype).

This invention, therefore, is directed to uses and methods of using the compositions against mitochondrial fragmentation as defined in the appended set of claims. The compositions used in the claimed methods and uses protect the skin from mitochondrial fragmentation, maintain or reduce reactive oxygen species, thereby preventing premature skin aging.

### Additional Information

Nicotinamide, also known as Niacinamide or a form of Vitamin B3, is well known in the art and is commercially available from sources including Sigma-Aldrich Chemical Company (St. Louis, Missouri, USA). However, its ability to increase ATP in cells is questionable, and it is believed to decrease ATP levels, hence lowering the overall bioenergetic state of the cell.

US-A-5939082 discloses topical compositions based on vitamin B3 for regulating the signs of skin ageing. US-A-4956173 and US-A-2004/213819 are directed to the use of SAM-e in anti-ageing cosmetic compositions.

Topical compositions aimed at reducing signs of aging using vitamin B are disclosed in US2007/0110731. NZ 526350 A discloses anti-aging compositions comprising an amino acid, vitamin B complex and preferably further including
S-adenosyl-L-methionine.

S-adenosyl-L-methionine (SAMe) is naturally occurring in the body and used as an oral supplement in humans. SAMe is a key participant in energy metabolism, playing a main role to transfer methyl groups to nucleic acids, proteins, lipids and secondary metabolites. SAMe plays a key role in polyamine biosynthesis, cellular growth and repair, maintaining proper cell membranes, breaking down brain chemicals, gene expression and immune system functioning.

Topical pharmaceutical compositions comprising SAMe are disclosed in Schaller et al. US 2006/0069059 A1 for treating depression.

Overall, none of the additional information has demonstrated topical cosmetic composition or method of applying the composition on skin containing SAMe to be effective for mitochondrial fragmentation. None of the additional information above discloses the mechanism of mitochondrial fragmentation. Furthermore, none of the additional information describes a composition with the skin benefit agent S-adenosyl-L-methionine (SAMe), in combination with niacinamide (B3) as decreasing mitochondrial fragmentation and/or reducing oxidative stress in skin cells when topically applied to the skin in a cosmetically suitable carrier.

### Summary of the invention

The present invention overcomes the prior art deficiencies by providing non-therapeutic methods and uses as defined in the appended set of claims for delivering consumer skin anti-aging and skin anti-stress benefits by protecting mitochondria and/or lowering mitochondrial fragmentation in skin cells.

Without wishing to be bound by theory, Applicants believe that mitochondrial fragmentation levels on skin cells may be used as a biomarker for boosting skin cellular energy. Compounds that protect the skin cells from mitochondrial fragmentation contribute to the increase in cellular energy required to maintain or improve the integrity and function of the skin. A lower number of fragmented mitochondria inside the cells allow for a healthier mitochondrial network that is associated with skin benefits (young skin phenotype).

The present invention is based on discovery of active ingredients for incorporation in topical compositions, which can deliver consumer skin benefits by lowering mitochondrial fragmentation levels in skin cells.

In a first aspect, the present invention is a non-therapeutic method for delivering consumer skin anti-aging and skin anti-stress benefits by protecting mitochondria and/or lowering mitochondrial fragmentation in skin cells of an individual in need thereof by topically applying to the skin a topical personal care composition
comprising: from 0.001 to 10%, preferably, from 0.01 to 6%, and most preferably, from
0.05 to 3.5%, niacinamide (B3) compound; and
from 0.001 to 10%, preferably from 0.01 to 6%, of SAMe;
in a cosmetically acceptable vehicle.

SAMe is a compound of **Structural Formula 1,** below:

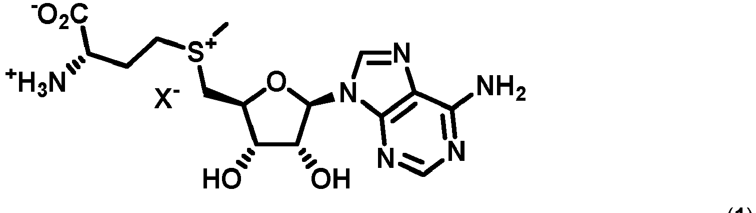

The composition may include additional optional skin benefit agents. compositions may optionally include additional skin benefit agents.

In a second aspect, the present invention is the non-therapeutic use of a topical personal care composition comprising from 0.001 to 10% niacinamide and from 0.001 to 10% by weight of S-adenosyl-L-methionine for delivering consumer skin anti-aging and skin anti-stress benefits by protecting mitochondria and/or lowering mitochondrial fragmentation in skin cells.

In a third aspect, the present invention is the non-therapeutic topical use of niacinamide and S-adenosine-L-methionine for delivering consumer skin anti-aging and skin anti-stress benefits by protecting mitochondria and/or lowering mitochondrial fragmentation in skin cells.

All other aspects of the present invention will readily become apparent upon considering the detailed description and examples which follow.

Throughout the specification and claims, the following terms take the meanings explicitly associated herein, unless the context clearly dictates otherwise.

The examples given in connection with the various embodiments of the invention are intended to be illustrative.

"Skin," as used herein, is meant to include skin on the feet, face (including oral cavity), neck, chest, back, arms, hands, legs, buttocks and scalp (including hair) of an individual.

In specifying any range of concentration or amount, any particular upper concentration can be associated with any particular lower concentration or amount.

"Comprising" is intended to mean "including" but not necessarily "consisting of" or "composed of." In other words, the listed steps or options need not be exhaustive. The term comprises is meant to encompass the terms consisting essentially of and consisting of.

The composition of this invention includes creams, lotions, balms, serums, deodorants and antiperspirants, shampoos, conditioners, bars and liquid wash products. In a preferred embodiment, the composition of this invention is a topical leave-on composition like a leave-on cream or lotion.

"Leave-on composition" refers to a composition that is applied to the skin and is not intended to be washed or rinsed off for some period of time, specifically hours, as contrasted with skin cleansing or wash-off or rinse-off compositions which are rinsed off or washed off immediately or minutes after the application. Both leave-on compositions and wash/rinse-off compositions are within the scope of "personal care compositions."

"Personal care composition" refers to any product applied to a human body for improving appearance, sun protection, cleansing (including oral care), odor control, moisturization or general aesthetics. Non-limiting examples of personal care compositions include skin lotions, creams, gels, lotions, facial masks, sticks, shampoos, conditioners, shower gels, toilet bars, antiperspirants, deodorants, shave creams, depilatories, sunless tanners and sunscreen lotions.

"Skin cosmetic composition" refers to any product applied to a human body for improving appearance, sun protection, reducing wrinkled appearance or other signs of photoaging, odor control, skin lightening, even skin tone, or general aesthetics. The composition of the invention which is suitable to provide benefit to skin can be an emulsion or a composition that is free of water and emulsifier. Non-limiting examples of topical cosmetic skin compositions include skin lotions, creams, facial masks, gels, sticks, antiperspirants, deodorants, liquid or gel body washes, soap bars, oral care products, sunless tanners and sunscreen lotions.

Using "against" as it refers to mitochondrial fragmentation includes but is not limited to protecting/offering protection, lowering, maintaining, preventing, and particularly in UV exposed skin.

### Detailed description of the invention

The present invention is directed to non-therapeutic methods and uses as defined in the appended set of claims.

The present invention is based on discovery of active ingredients for incorporation in topical compositions, which can deliver consumer skin benefits by protecting mitochondria and/or lowering mitochondrial fragmentation in skin cells. These compounds are associated with efficient use of energy, control of glucose and lipid metabolism, increase of cellular repair/rejuvenation and increase of anti-oxidant benefit. The personal care benefits of applying the cosmetic compositions include, without limitation, anti-ageing and anti-stress (including UV and oxidative stresses).

In a first aspect, the present invention is a method for delivering consumer skin anti-aging and skin anti-stress benefits by protecting mitochondria and/or lowering mitochondrial fragmentation in skin cells of an individual in need thereof by topically applying to the skin a topical personal care composition comprising
from 0.001 to 10% niacinamide (B3) compound and from 0.001 to 10%, preferably from 0.01 to 6%, of SAMe in a cosmetically acceptable vehicle.

SAMe is a compound of Structural Formula 1: 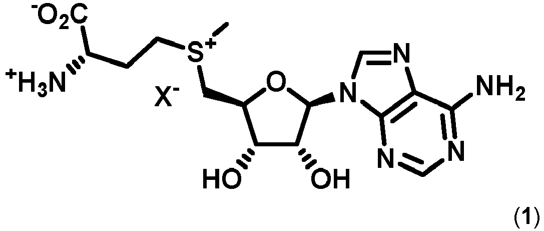 in the combination with niacinamide of Structural Formula 2:

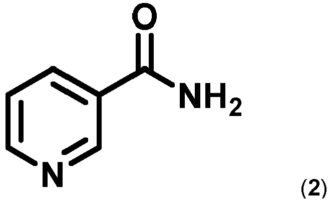

In a second aspect, the present invention is a non-therapeutic use of a topical personal care composition comprising from 0.001 to 10% niacinamide and from 0.001 to 10% by weight of S-adenosyl-L-methionine for delivering consumer skin anti-aging and skin anti-stress benefits by protecting mitochondria and/or lowering mitochondrial fragmentation in skin cells.

In a third aspect, the present invention is the non-therapeutic topical use of niacinamide and S-adenosine-L-methionine for delivering consumer skin anti-aging and skin anti-stress benefits by protecting mitochondria and/or lowering mitochondrial fragmentation in skin cells.

### S-Adenosyl-L-methionine ("SAMe")

According to the present invention, S-adenosyl-L-methionine compounds (Structural Formula 1 shown below) are used in combination with Niacinamide.

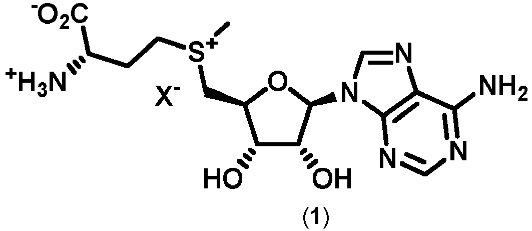

Typically, the amount of S-adenosyl-L-methionine (SAMe) used in the methods and uses of this invention is from 0.001 to 10%, and preferably, from 0.01 to 6%, and most preferably, from 0.05 to 3.5%, based on total weight of the composition and including all ranges subsumed therein.

### Nicotinamide

*Nicotinamide, also known as Niacinamide or a form of Vitamin B3,* has the following Structural Formula 2:

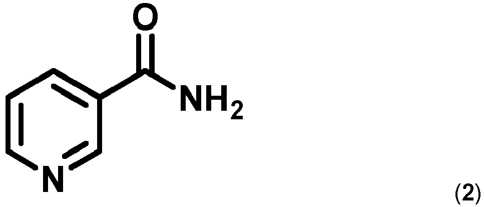

Typically, the amount of nicotinamide used in the methods and uses of this invention is from 0.001 to 10%, and preferably, from 0.01 to 6%, and most preferably, from 0.05 to 3.5%, based on total weight of the composition and including all ranges subsumed therein.

Advantageously and unexpectedly, the combinations of SAMe + B3 demonstrate the ability to lower mitochondrial fragmentation in skin cells.

### Salt Forms

Compounds of the present invention (SAMe and nicotinamide) are capable of forming a salt with a variety of physiologically suitable counterions, including, without limitation, chloride, bromide, iodide, hydroxide, sulfate, sulfonate, nitrate, phosphate, formate, tartrate, lactate, oxalate, fumarate, maleate, succinate, malonate, citrate or R₅CO₂⁻ where R₅ is a C₁-C₂₂ alkyl group, which may be linear, branched or cyclic, saturated or unsaturated, and substituted with one or more heteroatoms selected from O, S.

### Carrier

The compositions used in this invention can have as cosmetically acceptable carriers non-polar liquids like oils. Alternatively, such non-polar liquids can be used as the oil phase when the composition is an emulsion.

When the compositions used in the present invention are emulsions, they will typically include cosmetically acceptable carrier components iaddition to non-polar liquid. Water is the most preferred additional carrier. Amounts of water may range from 1 to 99%, and preferably, from 5 to 90%, and most preferably, from 35 to 80%, and optimally, from 40 to 75% by weight, based on total weight of the composition and including all ranges subsumed therein. Ordinarily the compositions used in this invention will be water and oil emulsions, most preferably, of the oil-in-water variety. Water-in-oil emulsions, and especially, those generally classified as water-in-oil and high internal phase emulsions are, however, an option. Illustrative examples of the high internal phase emulsions suitable to as carrier for this invention are described in commonly owned U.S. Patent Application Publication No. 2008/0311058 and U.S. Patent No. 8,425,882

Other cosmetically acceptable carriers suitable for use (with or without water) in this invention may include mineral oils, silicone oils, esters, and alcohols. Amounts of these materials may collectively range from 0.1 to 99%, and preferably, from 0.1 to 45%, and most preferably, from 1 to 20% by weight of the composition used in this invention, including all ranges subsumed therein.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, and preferably, from 4 to 5 silicon atoms.

Linear volatile silicone materials generally have viscosities of less than 5 mPa.s⁻¹ (centistockes) at 25°C. while cyclic materials typically have viscosities of less than about 10 mPa.s⁻¹ (centistokes)

Nonvolatile silicone oils useful as carrier material include polyalkyl siloxanes, polyalkylaryl siloxanes and polyether siloxane copolymers. The essentially non-volatile polyalkyl siloxanes useful herein include, for example, polydimethylsiloxanes (like dimethicone) with viscosities of from 5 to 100,000 mPa.s⁻¹ (centistokes) at 25°C.

A preferred silicone source is a cyclopentasiloxane and dimethiconol solution.

Among suitable esters are:
(l) Alkenyl or alkyl esters of fatty acids having 10 to 20 carbon atoms like isopropyl palmitate, isopropyl isostearate, isononyl isonanonoate, oleyl myristate, isopropyl myristate, oleyl stearate, and oleyl oleate;
(2) Ether-esters such as fatty acid esters of ethoxylated fatty alcohols;
(3) Polyhydric alcohol esters such as ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 mono stearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxy-ethylene sorbitan fatty acid esters;
(4) Wax esters such as beeswax, spermaceti, myristyl myristate, stearyl stearate; and
(5) Sterol esters, of which soya sterol and cholesterol fatty acid esters are examples thereof.

Often, oils such as caprylic capric triglyceride are preferred as carriers.

Emulsifiers may be present in the compositions used in the present invention. Total concentration of the emulsifier may range from about 0.1 to 40%, and preferably, from 1 to 20%, and most preferably, from 1 to 5% by weight of the composition, including all ranges subsumed therein. The emulsifier may be selected from the group consisting of anionic, nonionic, cationic and amphoteric actives. Particularly preferred nonionic actives are those with a C₁₀-C₂₀ fatty alcohol or acid hydrophobe condensed with from about 2 to about 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di-C₈-C₂₀ fatty acids; and polyoxyethylene sorbitan as well as combinations thereof. Alkyl polyglycosides and saccharide fatty amides (e.g. methyl gluconamides) are also suitable nonionic emulsifiers.

Preferred anionic emulsifiers include alkyl ether sulfate and sulfonates, alkyl sulfates and sulfonates, alkylbenzene sulfonates, alkyl and dialkyl sulfosuccinates, C₈-C₂₀acyl isethionates, C₈-C₂₀ alkyl ether phosphates, alkyl ether carboxylates and combinations thereof.

Cationic emulsifiers that may be used include, for example, almitamidopropyltrimonium chloride, distearyldimonium chloride and mixtures thereof. Useful amphoteric emulsifiers include cocoamidopropyl betaine, C₁₂-C₂₀ trialkyl betaines, sodium lauroamphoacetate, and sodium laurodiamphoacetate or a mixture thereof.

Other generally preferred emulsifiers include glyceryl stearate, glycol stearate, stearamide AMP, PEG-100 stearate, cetyl alcohol as well as emulsifying/thickening additives like hydroxyethylacrylate/sodium acryloyldimethyl taurates copolymer/squalane and mixtures thereof.

### Composition

Preservatives can desirably be incorporated into the compositions used in this invention to protect against the growth of potentially harmful microorganisms. Suitable traditional preservatives for compositions used in this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate, phenoxyethanol, 1,2-octanediol, ethylhexylglycerine, hexylene glycol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the composition, including all ranges subsumed therein. Combinations of 1,2-octanediol and phenoxyethanol, or iodopropynyl butyl carbamate and phenoxyethaol are preferred, with phenoxyethanol making up from 35 to 65% by weight of the total weight of the preservative combination with the phenoxyethanol.

Thickening agents may optionally be included in compositions used in the present invention.

Particularly useful are the polysaccharides. Examples include starches, natural/synthetic gums and cellulosics. Representative of the starches are chemically modified starches such as sodium hydroxypropyl starch phosphate and aluminum starch octenylsuccinate. Tapioca starch is often preferred. Suitable gums include xanthan, sclerotium, pectin, karaya, arabic, agar, guar, carrageenan, alginate and combinations thereof. Suitable cellulosics include hydroxypropyl cellulose, hydroxypropyl methylcellulose, ethylcellulose and sodium carboxy methylcellulose. Synthetic polymers are yet another class of effective thickening agent. This category includes crosslinked polyacrylates such as the Carbomers, polyacrylamides such as Sepigel 305 and taurate copolymers such as Simulgel EG and Arlstoflex AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100.

Amounts of the thickener, when used, may range from 0.001 to 5%, and preferably, from 0.1 to 2%, and most preferably, from 0.2 to 0.5% by weight of the composition and including all ranges subsumed therein.

Fragrances, fixatives and abrasives may optionally be included in compositions used in the present invention. Each of these substances may range from about 0.05 to about 5%, preferably between 0.1 and 3% by weight.

To enhance skin moisturization, cationic ammonium compounds may optionally be used in the compositions used in this invention to enhance moisturization. Such compounds include salts of hydroxypropyltri (C₁-C₃ alkyl) ammonium mono-substituted-saccharide, salts of hydroxypropyltri (C₁-C₃ alkyl) ammonium mono-substituted polyols, dihydroxypropyltri (C₁-C₃ alkyl) ammonium salts, dihydroxypropyldi (C₁-C₃ alkyl) mono(hydroxyethyl) ammonium salts, guar hydroxypropyl trimonium salts, 2,3-dihydroxypropyl tri (C₁-C₃ alkyl or hydroxalkyl) ammonium salts or mixtures thereof. In a most preferred embodiment and when desired, the cationic ammonium compound employed in this invention is the quaternary ammonium compound 1,2-dihydroxypropyltrimonium chloride. If used, such compounds typically make up from 0.01 to 30%, and preferably, from 0.1 to 15% by weight of the composition. When cationic ammonium compounds are used, additional preferred additives for use with the same are moisturizing agents such as substituted ureas like hydroxymethyl urea, hydroxyethyl urea, hydroxypropyl urea; bis(hydroxymethyl) urea; bis(hydroxyethyl)urea; bis(hydroxypropyl)urea; N, N'-dihydroxymethyl urea; N, N'-dihydroxyethyl urea; N, N'-dihydroxypropyl urea; N, N, N'-tri-hydroxyethyl urea; tetra (hydroxymethyl)urea; tetra(hydroxyethyl)urea; tetra (hydroxypropyl)urea; N-methyl-N'-hydroxyethyl urea; N-ethyl-N, N-N'-hydroxyethyl urea; N-hydroxypropyl-N'-hydroxyethyl urea and N, N'-dimethyl-N-hydroxyethyl urea or mixtures thereof. Where the term hydroxypropyl appears, the meaning is generic for either 3-hydroxy-n-propyl, 2-hydroxy-n-propyl, 3-hydroxy-i-propyl or 2-hydroxy-i-propyl radicals. Most preferred is hydroxyethyl urea. The latter is available as a 50%aqueous liquid from the National Starch & Chemical Division of ICI under the trademark Hydrovance.

Amounts of substituted urea, when used, in the composition used in this invention range from 0.01 to 20%, and preferably, from 0.5 to 15%, and most preferably, from 2 to 10% based on total weight of the composition and including all ranges subsumed therein.

Conventional humectants may be employed in the present invention as skin benefit agent and in addition to the bioenergetic combinations hereof. These are generally polyhydric alcohol type materials. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. Most preferred is glycerin, propylene glycol or a mixture thereof. The amount of humectant employed may range anywhere from 0.5 to 20%, preferably between 1 and 15% by weight of the composition.

When cationic ammonium compound and substituted urea are used, in a most especially preferred embodiment at least from 1 to 15% glycerin is used, based on total weight of the composition and including all ranges subsumed therein.

Compositions used in the present invention may optionally and additionally include vitamins, along with the actives, skin benefit agents, and/or derivatives thereof according to the present invention. Illustrative vitamins are retinol (Vitamin A), Vitamin B₂, Vitamin B₆, Vitamin C, Vitamin E, Folic Acid and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed and Vitamin D and K are also options. Total amount of optional vitamins when present in compositions according to the present invention may range from 0.0 to 10%, preferably from 0.001 to 1%, optimally from 0.01 to 0.5% by weight of the composition.

### Optional Skin Benefit Agents

The compositions used in the present invention can comprise in addition to niacinamide, skin benefit agents, and/or derivatives thereof, additional optional skin benefit agents (SBAs). It is preferred that to niacinamide, skin benefit agents, and/or derivatives thereof make up at least 25% by weight, and preferably, at least 40 to 95% by weight, and most preferably, 100% by weight of the skin benefit agents. Optional skin benefit agents or additives may, if desired, be provided to make up the portion of the skin benefit agent that is not niacinamide or cooperative skin benefit agent and/or a derivative thereof.

Also optionally suitable for use include materials like chelators (e.g., EDTA), opacifiers (like TiO₂, particle size from 50 to 1200nm, and preferably, 50 to 350nm), C₈₋₂₂ fatty acid substituted saccharides, lipoic acid, retinoxytrimethylsilane (available from Clariant Corp. under the SilCare IM-75 trademark), dehydroepiandrosterone (DHEA) and combinations thereof. Ceramides (including Ceramide I, Ceramide 3, Ceramide 36 and Ceramide 6) as well as pseudoceramides may also be useful. Amounts of these materials may range from 0.000001 to 10%, preferably from 0.0001 to 1% by weight of the composition used in this invention.

Sunscreen actives may also be included in compositions used in the present invention.

Particularly preferred are such materials as ethylhexyl p-methoxycinnamate, available as Parsol MCX, Avobenzene, available as Parsol 1789 and benzophenone-3, also known as Oxybenzone. Inorganic sunscreen actives may be employed such as microfine titanium dioxide, octocrylene zinc oxide, polyethylene and various other polymers.

Amounts of the sunscreen agents when present may generally range from 0.1 to 30%, preferably from 0.5 to 20%, optimally from 0.75 to 10% by weight.

Conventional buffers/pH modifiers may be used. These include commonly employed additives like sodium hydroxide, potassium hydroxide, hydrochloric acid, citric acid and citrate/citric acid buffers. In an especially preferred embodiment, the pH of the composition used in this invention is from 4 to 8, and preferably, from 4.25 to 7.75, and most preferably, from 6 to 7.5, including all ranges subsumed therein.

The composition used in the present invention preferably is a leave-on skin lotion, cream, shampoo, conditioner, shower gel, antiperspirant, deodorant, depilatory, shave cream or toilet bar.

### Packaging

A wide variety of packaging can be employed to store and deliver the compositions used in this invention. Packaging is often dependent upon the type of personal care end-use. For instance, leave-on skin lotions and creams, shampoos, conditioners and shower gels generally employ plastic containers with an opening at a dispensing end covered by a closure. Typical closures are screw-caps, nonaerosol pumps and flip-top hinged lids. Packaging for antiperspirants, deodorants and depilatories may involve a container with a roll-on ball on a dispensing end. Alternatively, these types of personal care products may be delivered in a stick composition formulation in a container with propel repel mechanism where the stick moves on a platform towards a dispensing orifice. Metallic cans pressurized by a propellant and having a spray nozzle serve as packaging for antiperspirants, shave creams and other personal care products. Toilette bars may have packaging constituted by a cellulosic or plastic wrapper or within a cardboard box or even encompassed by a shrink wrap plastic film.

The following examples are provided to facilitate an understanding of the present invention.

### Examples

### Experimental Methods

### Materials and Cell Treatment

Test compounds (B3 and SAMe) were purchased from Sigma (St. Loius, MO). Cell culture growth media (Epilife media with calcium (Cat# MEPI500CA) and Epilife media supplement HKGS 100X (Cat# S-001-5)) was purchased from Thermofisher (Waltham, MA). Cryopreserved human keratinocytes cells (~1 ×10⁶ cells) were plated on 12-well glass bottom plates at a confluence of -15%. The cells were suspended in primary keratinocyte growth media (Epilife media with calcium) and incubated @ 37 °C for 1d (Day 0). At Day 1, cells began treatment with supplemented primary keratinocyte growth media (Epilife media with calcium supplemented with HKGS 100X; 25ml total) without test compound (Control sample) or containing test compounds B3 (10mM), SAMe (500uM) or B3 (10mM) + SAMe (500uM). Cell treatment (25ml media with or without test compounds as above) continued daily for 2 additional days (Days 2 and 3). On Day 4, cells were subjected to UV treatment by replacing media with PBS, exposing cells to UV light (4J/cm² of UVA+UVB) for 14min and feeding cells with media with or without corresponding test compounds as defined above. UV treatments continued daily for 3 additional days (Days 5, 6 and 7). Image acquisition and analysis on all wells to assess mitochondrial fragmentation was conducted on Day 1, 3, 5 and 8.

### Image Acquisition

For two-photon excited fluorescence (TPEF) imaging, cell cultures were placed in microscope-compatible micro-incubator system, which maintained 37°C within a humidified environment throughout the imaging session. HEPES buffering agent (Sigma) was added to cell cultures before imaging, which maintained the pH of cell cultures throughout the imaging session. Images were obtained using a Leica TCS SP8 confocal microscope equipped with a tunable (680-1300 nm) titanium-sapphire laser (InSight Deep See; Spectra Physics; Mountain View, CA). Images (1024 x 1024 pixels; 386 x 386 µm) were acquired using water-immersion 25x objective (NA 0.95; 2.4 mm working distance), with simultaneous collection by two non-descanned photomultiplier tube (PMT) detectors using a filter cube containing filters from Chroma (Bellows Falls, VT), including a 700 nm short pass filter (ET700SP-2P) and a 495 nm dichroic mirror (495DCXR). To isolate NADH fluorescence, a 460(±20) nm emission filter (Chroma, ET460/40M-2P), corresponding to the NADH emission peak, was placed before one of the non-descanned detectors. NADH fluorescence images were acquired from this 460 nm channel using 755 nm excitation. FAD fluorescence was isolated using a 525(±25) nm emission filter (Chroma, ET525/50M-2P) for the other non-descanned detector and 860 nm excitation. The two-photon action cross section of NADH decreases by several orders of magnitude between 755 nm and 860 nm excitation, enabling an efficient isolation of FAD at longer wavelengths. The fluorescence lifetime images (512 x 512 pixels; 386 x 386 µm) corresponding to NADH were acquired under the same excitation and emission settings, using SymPho software. PMT gain was adjusted for each image to maximize contrast while preventing a saturated pixel intensity value. The PMT gain and laser power were recorded for each image and used to normalize fluorescence intensity.

### Mitochondrial Fragmentation Calculation

To assess mitochondrial fragmentation, a previously-established Fourier transform technique was used to obtain power spectral density (PSD) curves from each image. (M. Levitt et al., Diagnostic cellular organization features extracted from autofluorescence images. Opt Lett 32, 3305-3307 (2007); J. Xylas, K. P. Quinn, M. Hunter, I. Georgakoudi, Improved Fourier-based characterization of intracellular fractal features. Opt Express 20, 23442-23455 (2012). Briefly, the image intensity patterns within the cell cytoplasmic areas selected by the same binary mask used for the redox analysis were cloned and randomly positioned in the image background to create a new image without distinct cell borders and only cell mitochondrial patterns spanning the entire image (K. P. Quinn et al., Quantitative metabolic imaging using endogenous fluorescence to detect stem cell differentiation. Sci Rep 3, 3432 (2013). Upon Fourier transformation a power spectral density (PSD) curve was created for each image. An inverse power law behavior of the PSD curve at high spatial frequencies (> 0.1 µm-1, corresponding to the size of mitochondria) was then identified. This portion between 0.1 µm-1 and the frequency at 98% of the entire PSD region was then fitted with an equation of the form R(k)~ k-β and the exponential power, β, which represented the mitochondrial fragmentation throughout this study was determined.

### Statistical Analyses

To compare means between treatments on each day, JMP and Tukey HSD was used for the statistical analyses. The significance level of the test (α, the probability of a type I error) was set to 0.05. P-values </= to 0.05 between two measurements are considered statistically significant.

### EXAMPLE 1. Mitochondrial Fragmentation in keratinocytes - UV Study

The purpose of this experiment was to compare the extent of protection to skin delivered by different actives from mitochondrial fragmentation that takes place as a consequence of aging with or without UV exposure.

The higher the Mitochondrial Fragmentation value, the less protection to the skin is delivered by the given active or combination of actives. Note, for biological systems such as mitochondria, which are microscale components of skin cells, the absolute values of mitochondrial fragmentation numbers are small, and small differences can be significant.

The effect of B3 and low levels of SAMe on mitochondrial fragmentation in keratinocytes 3 days before and 4 days after UV-exposure is shown in the Table below:

**TABLE 1**

| **Test Sample** | **Mitochondrial Fragmentation** | |
|---|---|---|
| | **(-) UV (3 days)** | **(+) UV (4 days)** |
| Control (no treatment) | 1.30 | 1.58 ^{c} |
| B3 (10mM) | 1.18 | 1.34 ^{d} |
| SAMe (500uM) | 1.30 | 1.58 ^{c,e} |
| SAMe (500uM) + B3 (10mM) | 1.22 | 1.16 ^{a,b,d,f} |

| | | |
|---|---|---|
| ^{a} Significantly better over Non UV-exposed control (P </= 0.05) ^{b} Significantly better over B3 alone (P </= 0.05) ^{c} Significantly worse over Non-UV exposed control (P </= 0.05) ^{d} Significantly better over UV-exposed control (P </= 0.05) ^{e} Significantly worse over B3 (P </= 0.05) ^{f} Significantly better over SAMe alone (P </= 0.05) | | |

As can be seen from the data in Table 1 above, treatment of Non UV-exposed keratinocytes with high levels of B3 (10mM), low levels of SAMe (500uM) or a combination of low levels of SAMe (500uM) + high levels of B3 (10mM) does not significantly reduce mitochondrial fragmentation. UV exposure alone for four (4) days is effective at raising the control levels of mitochondrial fragmentation. While B3 alone at elevated doses (10mM) is effective at reducing this UV-induced effect down to control levels, SAMe alone at much lower doses (500uM) increases mitochondrial fragmentation beyond UV-exposed levels. However unexpectedly, the combination of B3 (10mM) with small amounts (20-fold less) of SAMe (500uM) effectively and synergistically lowers the mitochondrial fragmentation over either B3 or SAMe treatment alone and beyond Non UV-exposed control levels, hence, offering superior cooperative benefits.

### EXAMPLE 2

Personal care formulations used in the present invention are illustrated in the Tables below. All numbers in Tables represent weight % in the composition.

**TABLE 2A - Oil-in-water formulations, lotions, and creams**

| | OW-1 | OW-2 | OW-3 | OW-4 | OW-5 |
|---|---|---|---|---|---|
| Water | To 100 | To 100 | To 100 | To 100 | To 100 |
| Glycerine | 0-40 | 1-40 | 1-5 | 1-10 | 1-40 |
| Propylene glycol | 0-5 | | 0-5 | | |
| Butylene glycol | 0-5 | | 0-5 | 0-5 | |
| Carbomer | 0-2 | 0.03-1 | | | |
| Ammonium Acryloyl dimethyl taurate/VP copolymer | 0-1 | | 0.03-1 | | 0.01-1 |
| Styrene/Acrylates copolymer | 0-1 | | 0.01-1 | | |
| Xanthan Gum | 0-1 | | | | 0.01-1 |
| EDTA | 0.01-0.01 | 0.01-0.01 | 0.01-1 | 0.01-1 | 0.01-1 |
| Preservative | 0.02-2 | 0.02-2 | 0.02-2 | 0.02-2 | 0.02-2 |
| Titanium oxide | 0-10 | 0.01-10 | 0.01-10 | 0.01-10 | 0.01-10 |
| Colorant/Pigment | 0-5 | 0-5 | 0-5 | 0-5 | 0-5 |
| Triethanol amine /Sodium Hydroxide / potassium Hydroxide | 0-3 | 0.01-3 | 0.01-3 | 0.01-3 | 0.01-3 |
| Stearic acid | 0-5 | 0.01-5 | 0.01-5 | 0.01-5 | 0.01-5 |
| Isopropyl Myristate | 0-10 | 0.01-10 | | | |
| Capric/Caprylic Triglyceride | 0-10 | 0.01-10 | | | |
| C12-C15 alkyl benzoate | 0-10 | | | | 0.01-10 |
| Mineral oil | 0-10 | | | 0.01-10 | |
| Glyceryl stearate | 0-5 | 0.01-5 | | | |
| Steareth-2 | 0-5 | | 0.01-5 | | 0.01-5 |
| Steareth-21 | 0-5 | | 0.01-5 | | |
| Peg100 Stearate | 0-5 | | | 0.01-2 | 0.01-5 |
| Potassium Cetyl Phosphate | 0-5 | | | 0.01-2 | |
| Tween20 | 0-5 | | | | 0.01-5 |
| Cetyl alcohol | 0-4 | 0.01-4 | | 0.01-4 | |
| Dicaprylyl carbonate | 0-5 | | 0.01-5 | | |
| UVA and/or UVB Sunscreens | 0-6 | 0.01-6 | | 0.01-10 | 0.01-10 |
| Silicones | 0-15 | 0.01-10 | 0.01-15 | | |
| B3 | 1-5 | 0.01-10 | 0.01-10 | 0.01-10 | 0.01-10 |
| S-adenosylmethionine (SAMe) | 0.01-10 | 0.01-2 | 0.01-5 | 0.01-5 | 0.01-7 |

**TABLE 2B - Water-in-oil topical lotions or creams**

| | WO-1 | WO-2 | WO-3 | WO-4 |
|---|---|---|---|---|
| Water | To 100 | To 100 | To 100 | To 100 |
| Glycerine | 0-70 | 1-70 | 1-70 | |
| Propylene glycol | 0-5 | | | 0.01-5 |
| Butylene glycol | 0-5 | | 0.01-5 | 0.01-5 |
| Disteardimonium Hectorite | 0.01-1 | 0.01-1 | | |
| EDTA | 0.01-.01 | 0.01-1 | 0.01-1 | 0.01-1 |
| Preservative | 0.02-2 | 0.02-2 | 0.02-2 | 0.02-2 |
| TiO2 | 0-10 | 0.01-10 | 0.01-10 | 0.01-10 |
| Colorant/pigment | 0-5 | 0-5 | 0-5 | 0-5 |
| TEA/Sodium Hydroxide/potassium Hydroxide | 0-3 | 0.01-3 | 0.01-3 | 0.01-3 |
| Stearic acid | 0-5 | 0.01-5 | | |
| Isopropyl Myristate | 0-10 | | | |
| Capric/Caprylic Triglyceride | 0-10 | | 0.01-10 | |
| C12-C15 alkyl benzoate | 0-10 | | | 0.01-10 |
| Mineral oil | 0-10 | | | |
| Glyceryl stearate | 0-5 | | | |
| Dimethicone copolyol | 0-5 | 0.01-5 | 0.01-5 | |
| Cetyl PEG/PPG-10/1 Dimethicone | 0-5 | | | 0.01-5 |
| Steareth-2 | 0-2 | | | |
| Sucrose Distearate | 0-2 | 0.01-2 | | |
| Cetyl alcohol | 0-2 | 0.01-2 | 0.01-2 | |
| UVA and/or UVB Sunscreens | 0-6 | 0.01-6 | 0.01-10 | 0.01-10 |
| Dimethicone | 0-10 | | 0.01-10 | 0.01-10 |
| Cyclomethicone | 0-40 | 0.01-40 | | 0.01-10 |
| Caprylyl methicone | 0-10 | 0.01-10 | | 0.01-10 |
| Dimethicone crosspolymer | 0-90 | 0.01-90 | 0.01-90 | |
| C30-C45 alkyl cetearyl dimethicone crosspolymer | | | | 0.01-90 |
| Glycolic acid | 0-10 | 0.01-10 | | |
| KCI | 0-5 | 0.01-5 | 0.01-5 | 0.01-5 |
| Nicotinamide (B3) | 0.001-10 | 0.01-10 | 0.01-10 | 0.01-10 |
| S-adenosylmethionine (SAMe) | 0.01-2 | 0.01-2 | 0.01-1 | 0.01-5 |

**TABLE 2C - Vanishing Creams**

| | VC1 | VC2 | VC3 | VC4 |
|---|---|---|---|---|
| Water | To 100 | To 100 | To 100 | To 100 |
| Glycerine | 0-5 | 0.01-5 | 0.01-5 | |
| EDTA | 0.01-.01 | 0.01-.01 | 0.01-.01 | 0.01-.01 |
| Preservative | 0.02-2 | 0.02-2 | 0.02-2 | 0.02-2 |
| TiO2 | 0.01-10 | 0.01-10 | 0.01-10 | 0.01-10 |
| Colorant/pigment | 0-5 | 0.01-5 | 0.01-5 | |
| EA/Sodium Hydroxide/potassium Hydroxide | 0-3 | 0.01-3 | 0.01-3 | 0.01-3 |
| Stearic acid | 0-30 | 0.01-30 | 0.01-30 | 0.01-30 |
| Isopropyl Myristate | 0-5 | 0.01-10 | 0.01-10 | |
| C12-C15 alkyl benzoate | 0-5 | | | 0.01-10 |
| Brij 35 | 0-5 | 0.01-5 | | |
| Tween40 | 0-5 | | | 0.01-5 |
| Cetyl alcohol | 0-2 | 0.01-2 | 0.01-2 | |
| Ethyl hexyl methoxycinnamate | 0-6 | 0.01-6 | 0.01-6 | |
| Butyl Methoxydibenzoylmethane | 0-3 | 0.01-3 | 0.01-3 | 0.01-3 |
| Ensulizole | 0-4 | | | 0.01-4 |
| Octisalate | 0-5 | | | 0.01-5 |
| Octocrylene | 0-10 | | 0.01-10 | 0.01-10 |
| Dimethicone | 0-5 | 0.01-5 | | |
| Cyclomethicone | 0-5 | | | 0.01-5 |
| Dimethicone crosspolymer | 0-4 | | | 0.01-4 |
| Hydroxystearic acid | 0-5 | 0.01-5 | 0.01-5 | 0.01-5 |
| Fragrance | 0-2 | 0-2 | 0-2 | 0-2 |
| Nicotinamide (B3) | 0.01 -3 | 0.01 -3 | 0.01 -3 | 0.01 -3 |
| S-adenosylmethionine (SAMe) | 0.01-3 | 0.01-3 | 0.01-1 | 0.01-5 |

## Claims

1. A non-therapeutic method for delivering consumer skin anti-aging and skin anti-stress benefits by protecting mitochondria and/or lowering mitochondrial fragmentation in skin cells of an individual in need thereof by topically applying to the skin a topical personal care composition for improving energy efficiency of skin cells, comprising:
(a) from 0.001 to 10% preferably, from 0.01 to 6%, and most preferably, from 0.05 to 3.5%, by weight, of niacinamide; and
(b) from 0.001 to 10%, preferably from 0.01 to 6%, by weight, of S-adenosyl-L-methionine compound of Structural Formula 1: 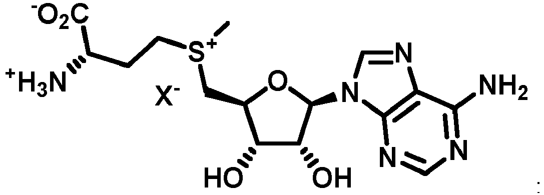
(c) an optional additional skin benefit agent; and
(d) a cosmetically acceptable carrier.

2. The non-therapeutic method according to claim 1 which improves cellular energy efficiency by protecting the skin of an aging individual in need thereof from mitochondrial fragmentation.

3. Non-therapeutic use of a topical personal care composition comprising from 0.001 to 10% niacinamide and from 0.001 to 10% by weight of S-adenosyl-L-methionine for delivering consumer skin anti-aging and skin anti-stress benefits by protecting mitochondria and/or lowering mitochondrial fragmentation in skin cells.

4. Non-therapeutic topical use of niacinamide and S-adenosine-L-methionine for delivering consumer skin anti-aging and skin anti-stress benefits by protecting mitochondria and/or lowering mitochondrial fragmentation in skin cells.

## Patentansprüche

1. Nicht-therapeutisches Verfahren zur Verschaffung von Antiaging- und Antistress-Vorteilen für die Haut eines Verbrauchers durch Schutz der Mitochondrien und/oder durch Verringerung der mitochondrialen Fragmentierung in den Hautzellen einer Person, die dies benötigt, durch topisches Auftragen einer topischen Pflegezusammensetzung auf die Haut zur Verbesserung der Energieeffizienz von Hautzellen, umfassend:
(a) 0,001 bis 10 Gewichts-%, bevorzugt 0,01 bis 6 Gewichts-% und höchst bevorzugt 0,05 bis 3,5 Gewichts-% Niacinamid; und
(b) 0,001 bis 10 Gewichts-%, bevorzugt 0,01 bis 6 Gewichts-% S-Adenosyl-L-methionin-Verbindung der Strukturformel 1: 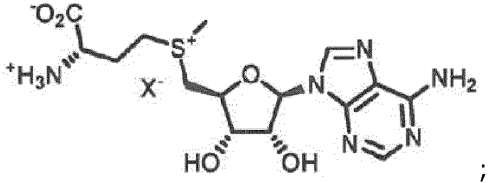
(c) ein optionales zusätzliches Hautpflegemittel; und
(d) einen akzeptablen kosmetischen Träger.

2. Nicht-therapeutisches Verfahren nach Anspruch 1, das die zellulare Energieeffizienz durch Schutz der Haut einer alternden Person, die dies benötigt, vor mitochondrialer Fragmentierung.

3. Nicht-therapeutische Verwendung einer topischen Pflegezusammensetzung, umfassend 0,001 bis 10 Gewichts-% Niacinamid und 0,001 bis 10 Gewichts-% S-Adenosyl-L-methionin zur Verschaffung von Antiaging- und Antistress-Vorteilen für die Haut eines Verbrauchers durch Schutz der Mitochondrien und/oder durch Verringerung der mitochondrialen Fragmentierung in den Hautzellen

4. Nicht-therapeutische topische Verwendung von Niacinamid und S-Adenosin-L-methionin zur Verschaffung von Antiaging- und Antistress-Vorteilen für die Haut eines Verbrauchers durch Schutz der Mitochondrien und/oder durch Verringerung der mitochondrialen Fragmentierung in den Hautzellen.

## Revendications

1. Procédé non thérapeutique pour délivrer des bénéfices antistress cutané et antivieillissement cutané à un consommateur par protection des mitochondries et/ou réduction de la fragmentation mitochondriale dans les cellules cutanées d'un individu en ayant besoin, par application topique à la peau d'une composition de soin personnel à usage topique pour améliorer le rendement énergétique de cellules cutanées, comprenant :
(a) de 0,001 à 10 %, de préférence de 0,01 à 6 % et tout spécialement de 0,05 à 3,5 % en poids de niacinamide ; et
(b) de 0,001 à 10 %, de préférence de 0,01 à 6 % en poids de composé S-adénosyl-L-méthionine de formule structurelle 1 : 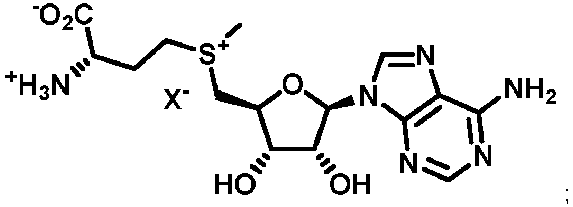
(c) un agent bénéfique pour la peau additionnel optionnel ; et
(d) un véhicule acceptable en cosmétique.

2. Procédé non thérapeutique selon la revendication 1, qui améliore le rendement énergétique cellulaire en protégeant la peau d'un individu vieillissant en ayant besoin vis-à-vis d'une fragmentation mitochondriale.

3. Utilisation non thérapeutique d'une composition de soin personnel à usage topique comprenant de 0,001 à 10 % de niacinamide et de 0,001 à 10 % en poids de S-adénosyl-L-méthionine pour délivrer des bénéfices antistress cutané et antivieillissement cutané à un consommateur par protection des mitochondries et/ou réduction de la fragmentation mitochondriale dans les cellules cutanées.

4. Utilisation topique non thérapeutique de niacinamide et de S-adénosine-L-méthionine pour délivrer des bénéfices antistress cutané et antivieillissement cutané à un consommateur par protection des mitochondries et/ou réduction de la fragmentation mitochondriale dans les cellules cutanées.
